# EUROPEAN PATENT SPECIFICATION

(11) **EP 1 927 318 B1**
(45) Date of publication and mention of the grant of the patent: **30.03.2016**
(21) Application number: 06810302.7
(22) Date of filing: 20.09.2006
(51) Int. Cl.: A61B 17/00, A61L 29/00, A61M 1/34

(54) **FILTER OF CATHETER FOR CAPTURING THROMBI AND METHOD OF PRODUCING THE SAME**
KATHETERFILTER ZUM ERKENNEN VON THROMBI UND HERSTELLUNGSVERFAHREN DAFÜR
FILTRE DE CATHÉTER POUR LA CAPTURE DE THROMBI ET PROCÉDÉ POUR LE PRODUIRE

(30) Priority: 22.09.2005 JP 2005274928
(43) Date of publication of application: 04.06.2008
(73) Proprietor: NIPRO CORPORATION, Kita-ku Osaka-shi, Osaka 531-8510 (JP)
(72) Inventor: ISSHIKI, Takaaki, Tokyo 1570066 (JP); KATAISHI, Yuichi, Yokohama-shi Kanagawa 2360032 (JP); MIYAGAWA, Katsuya, Osaka-shi Osaka 5318510 (JP); KATAOKA, Hideaki, Osaka-shi Osaka 5318510 (JP); UMEGAKI, Ryota, Osaka-shi Osaka 5318510 (JP)
(74) Representative: Vossius & Partner Patentanwälte Rechtsanwälte mbB
(86) International application number: PCT/JP2006/318606
(87) International publication number: WO 2007/034820

(56) References cited:
- WO-A1-01/74255
- WO-A1-2005/027751
- JP-A- 03 184 565
- JP-A- 11 319 102
- JP-A- 2004 097 807
- JP-A- 2004 097 807
- US-A1- 2002 004 667

## Description

### Technical Field

The present invention relates to a method producing a filter for thrombus capture catheters, which captures fragmentary thrombi released from a stenotic area of blood vessel such as coronary artery, carotid artery and a venous graft when performing percutaneous transluminal angioplasty at the stenotic area of blood vessels.

### Background Art

So far, percutaneous transluminal angioplasty such as stenting and POBA (Plain Old Balloon Angioplasty) has been applied to stenotic areas of blood vessels such as coronary arteries, carotid arteries and venous grafting. Such a procedure, which is one of revascularization procedures for securing the blood flow by performing stent placement or balloon dilution at the vascular stenotic area, has produced good results.

The above procedure enables to dilate the stenotic area, but it allows plaques or blood clots released from the diseased area by the procedure to flow in the peripheral blood vessels, causing infarction or No-Reflow phenomenon, which leads to failure in reperfusion. In most cases, complication caused by these blood clots produce severe symptoms.

For example, in case of unstable angina pectoris or acute myocardial infarction related to the blood clots in the coronary artery, the clots may be pushed out of the diseased area by stent placement or balloon dilation, resulting in blockage of blood vessels peripheral to the diseased area. This precludes adequate supply of oxygen and nutrients to the cardiac muscles, leading to myocardial failure such as myocardial necrosis. Similarly, blockage of the blood vessels supplying blood to the brain causes cerebral infarct, resulting in death of the brain tissues.

In particular, the carotid arteries are prone to stenosis because of arteriosclerosis, leading to insufficient blood flow to the brain or cerebral infarction resulting from the blood clots produced in the stenotic area. Symptoms of the cerebral infarction include paralysis, palsy, aphasia, consciousness disorder and the like. A patient does not foresee a 100 percent recovery of lost function even after receiving rehabilitative therapies. Thus, the patierit's life would be considerably affected. Further, caution should be exercised in patients with aging venous grafts since a great deal of clots are attached to the stenotic areas of the venous grafts.

As described above, the infarction caused by thrombi leads to serious complications and thus the percutaneous transluminal angioplasty is required to protect blood vessels on the downstream side of the diseased area. To this end, some inventions relating to a catheter with a thrombus capture filter have been applies for patents (For example, patent document 1).
[Patent document 1] JP 2004-97807A

JP 2004-97807 A relates to a thrombus capture catheter with a filter. The filter has pores with a size that prevents thrombus to path through to entrap it in the filter but allows a body fluid to pass therethrough. The filter may be produced by a dip coating method.

US 2002/0004667 A and WO 01/74255 represent another related art and describe that a filter may be produced by a dip coating method.

WO 2005/027751 discloses a thrombus collecting device with a bag-like filter.

### Disclosure of Invention

### Problems to be solved by the invention

The thrombus capture member of the above catheter is provided with a filter having pores. When introducing the thrombus capture member into the blood vessel, the thrombus capture member is loaded into a sheath under the contracted condition. Considering minimally invasive procedure and easy passage of the sheath through the diseased area in the blood vessel, the sheath is required to have a diameter as small as possible and, thus, the filter is required to have a thinner wall and to be firmly fixed to the cross wire assembly.

In the case of the above thrombus capture member, the filter is attached to the cross wire assembly by a method of wrapping a filter membrane around the cross wire assembly or a method of covering the cross wire assembly with the filter membrane. However, it is difficult with these methods to fix the filter to the cross wire assembly, reading to failure to obtain sufficient strength. In addition, there is a possibility that the filter membrane has to be thinned according to the method to be used with loss of the strength of fitter per se.

In view of these issues, an object of the present invention is to provide a method of producing a filter for thrombus capture catheters having a thin wall and a sufficient strength.

### Means for solving the problems

The above object is achieved with the features of claim 1.

The invention makes it possible to produce thin filters for thrombus capture catheters as the filtering section is composed of the cross wire assembly covered with the filter material.

Furthermore, the invention makes it easy to produce a filtering section with any predetermined size.

The wire may be of a shape-memory alloy and the filter material may be a biocompatible synthetic resin.

This makes it possible to produce a filter with any predetermined shape, which is harmless to humans and which is capable of holding the predetermined shape even if being deployed or contracted.

The inner pin and the core member may be provided with coatings of a lubricating material such as polytetra-fluoroethylene.

This makes it easy to perform attachment and removal of the inner pin and the core member and enable to remove these parts without damaging the filter membrane after formation of the filtering section.

According to the present invention, the filtering section is composed of the wire assembly coated with the filter material, thus making it possible to obtain a filter for thrombus capture catheters with a thin wall and sufficient strength.

### Brief Description of Drawings

Fig. 1 is a front elevation view of a wire assembly of a thrombus capture catheter;
Fig. 2 is a front elevation view illustrating the condition of an inner pin and a fixing core member being arranged in the inside of the wire assembly;
Fig. 3 is a front elevation view illustrating the conditiori of the wire assembly of Fig. 2 being set in a dipping device;
Fig. 4 is a front elevation view illustrating the condition of the wire assembly being dipped in a filter material solution;
Fig. 5 is a front elevation view illustrating the condition of the wire assembly being provided with a filter; and
Fig. 6 is an enlarged cross-section view along the line A-A in Fig. 5.

### Description of Symbols

1 wire
2 wire assembly
2A distal portion
2B proximal portion
2C middle portion
3 filtering section
4 inner pin
5 fixing core member
6 fixed assembly holder
10 fixed assembly
11 dipping apparatus

### Best Mode for Carrying out the Invention

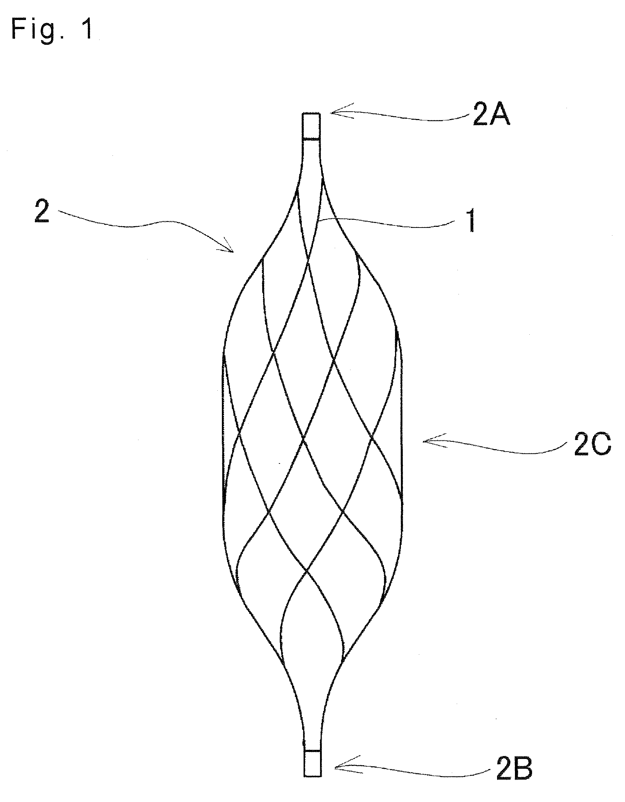

The filter for thrombus capture catheters and a method of producing the same according to the present invention will be explained below in detail with reference to the accompanying drawings of Figs. 1 to 5.

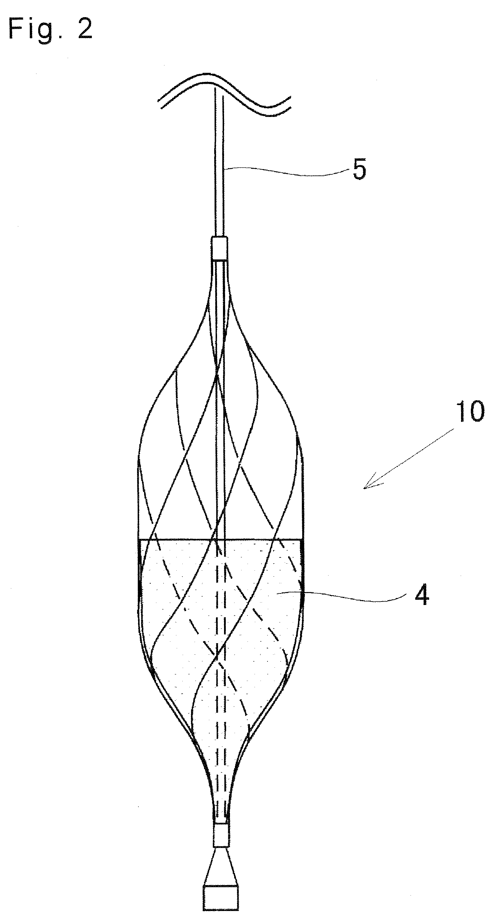

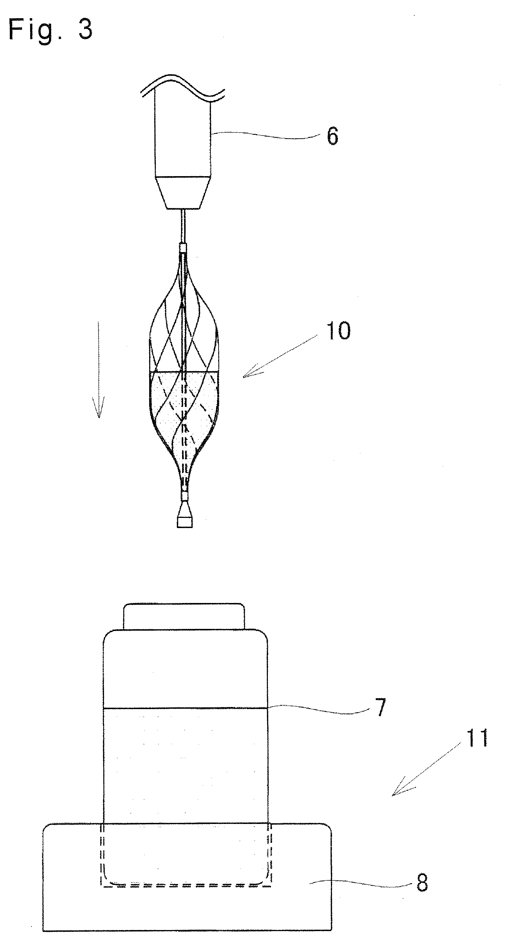

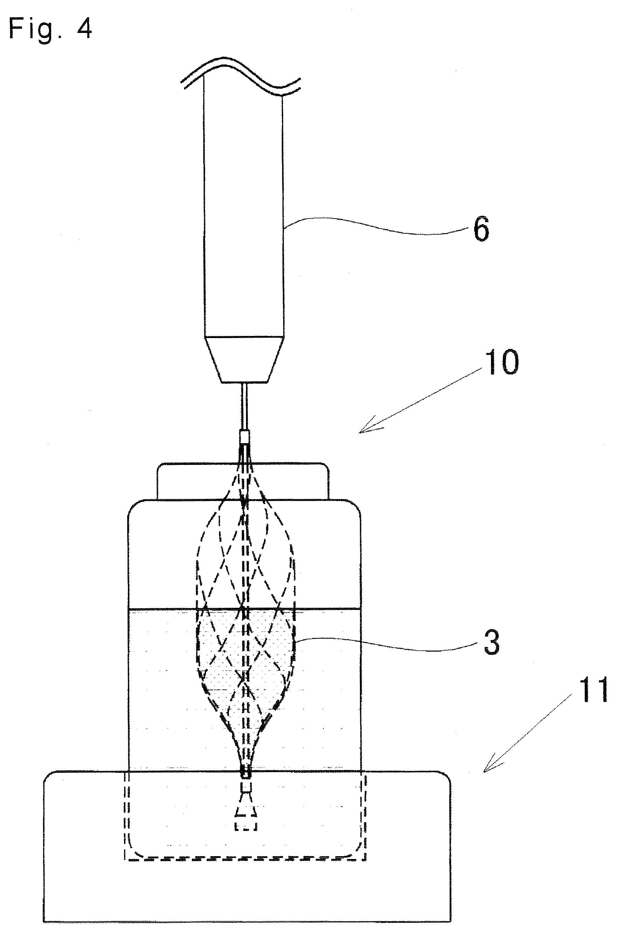

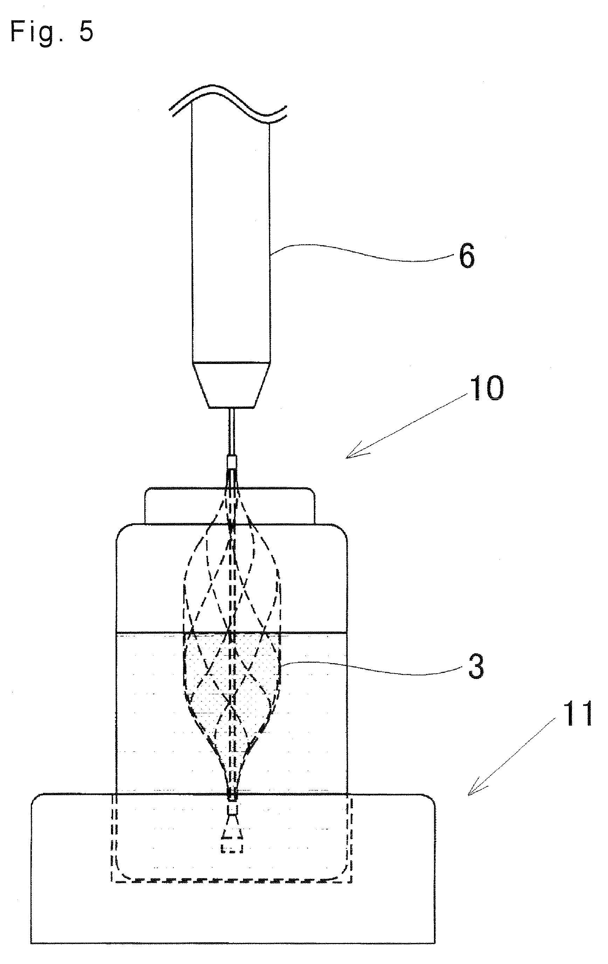

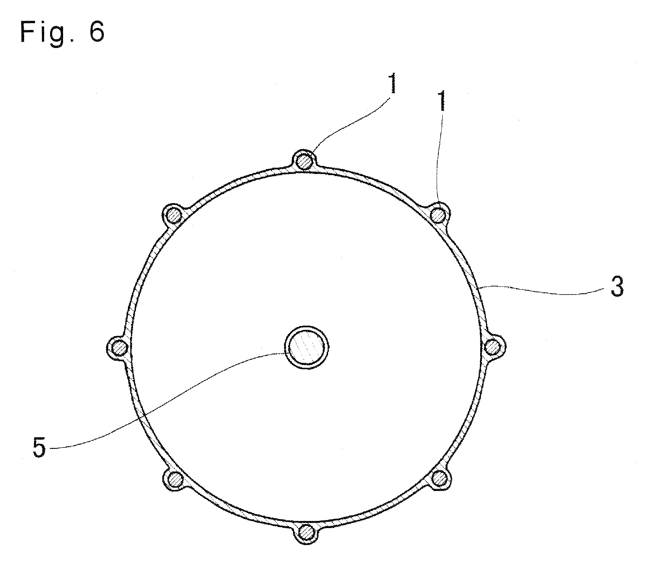

Fig. 1 is a front elevation view of a wire assembly of a thrombus capture catheter; Fig. 2 is a front elevation view illustrating the condition of an inner pin and a fixing core member being arranged in the inside of the wire assembly; Fig. 3 is a front elevation view illustrating the condition of the wire assembly of Fig. 2 being set in a dipping device; Fig. 4 is a front elevation view illustrating the condition of the wire assembly being dipped in a filter material solution; Fig. 5 is a front elevation view illustrating the condition of the wire assembly being provided with a filter; and Fig. 6 is an enlarged cross-section view along the line A-A in Fig. 5.

A filter for thrombus capture catheters according to the present invention is constructed by arranging a plurality of wires 1 so as to run helically, gathering and bundling them at both ends thereof to constitute a string bag-like wire assembly 2, which is converged at a distal portion 2A and a proximal portion 2B and which is expanded at a middle portion 2C, and then applying a filter material to about half of the wire assembly, which ranges from the middle portion 2C to one end (e.g., proximal portion 2B) of the wire assembly, by a dip coating process to form a filtering section covered with and fixed by the filter material.

Fig. 1 shows the wire assembly 2 constructed by arranging a plurality of wires 1 so as tao run helically. The wire assembly is so constructed freely stretchable and the bag-shaped portion covered with the filter material is deployable and foldable like a parachute canopy. In other words, there is provided a parachute canopy-like, deplorable and foldable filtering section 3 which is formed on about half of the wire assembly 2 ranging from the middle portion which serves as an opening, to one end of the wire assembly 2 (cf. Fig. 5).

The method of producing the above filters for thrombus capture catheters comprises firstly arranging a plurality of wires 1 so as to run helically, thereby forming a string bag-like wire assembly 2 which is converged at a distal portion 2A and a proximal portion 2B and which is expanded at an middle portion 2C; then inserting an inner pin 4, which serves as a parachute canopy-shaped framework, into an inside of the wire assembly, and inserting a core member into a central portion of the resultant wire assembly. This means the inner pin is provided with a central through-hole (not shown) which allows the core member to pass therethrough.

Thus, when the wire assembly 2 is made into a fixed assembly 10 with the inner pin 4 and core member 5 being inserted into the inside of the wire assembly 2 as shown in Fig. 2, the wire assembly has a fixed configuration, thus making it possible to immerse it as it is in a solution of a filter material contained in a container In other words, the fixed assembly 10 can be immersed in a dipping apparatus 11 while holding the fixed assembly 10 with a fixed assembly holder 6, as shown in Fig. 3.

The dipping apparatus 11 has a construction wherein a solution bottle containing a solution of the filter material is supported by a fixation device 8, and is provided with the vertically-movable fixed assembly holder 6. The fixed assembly 10, after being attached to the fixed assembly holder 6, is immersed in the solution of the filter material as shown in Fig. 4 by moving the fixed assembly holder 6 downward. The fixed assembly is dipped to a depth where the inner pin 4 arranged in the inside of the wire assembly is dipped in the solution to form a filtering section 3 having a configuration corresponding to the spindle-shaped external configuration of the inner pin 4. After completing the formation of the filtering section 3 with a desired configuration, the holder 6 is moved upward (cf Fig. 5) to dry the coated filter material. Thereafter, the inner pin 4 and the core member 5 are removed from the fixed assembly to complete the filter.

The filtering section 3 is formed under the condition that the spindle-shaped inner pin 4 and the core member 5 have been assembled in the wire assembly, so that the cross section of the filtering section becomes an annular shape covering a plurality of wires 1 as shown in Fig. 6. The filter material adheres to circumferences of the wires 1 much more than other portions and forms a coating with a configuration including a plurality of thick-walled portions in the circle, thus making it possible to obtain a filter with a thin wall thickness and a sufficient strength.

Materials that may be used for wires 1 are shape-memory alloys, and generally used materials are Ni-Ti alloys and Cu-Zn-Al alloys. In case shape memory alloys are used for production of wires, the wire assembly is generally formed so as to store a shape (returning original shape) as shown in Fig. 1. For the purpose of applying the filter to the diseased area with many blood clots, the wire assembly may be lengthened to make it possible to capture blood clots as much as possible. The number of wires constituting the wire assembly 2 ranges from 8 to 16, preferably from 8 to 14. The most preferable number of wires ranges from 8 to 12.

The wire assembly 2 may have, but is not limited to, an outer diameter of 3.0, 3.5, 4.0, 4.5, 5.0, 5.5, 6.0, 6.5, 7.0, 7.5, 8.0 and 12.0 mm and the wire assembly to be used is preferable determined according to the blood vessel size of the diseased area.

As a filter material, it is preferred to use a material which may turn into a solution allowing the cross wire assembly to be immersed therein. The preferred filter material includes biocompatible synthetic resins such as, for example, polyurethane, polyethylene, polyester, polypropylene, polytetrafluoroethylene, and polyvinylidenefluoride.

The inner pin 4 is a jig serving as a base for formation of filter membrane (a piece of a metal such as stainless steel or a piece of resin, coated with "Teflon (registered trademark)") and is capable of being placed into the wire assembly 2 through the gap between freelydisplaceable wires 1. In this case, the placement of the inner pin 4 causes no permanent deformation in the wire assembly 2 as the wires 1 return to their original shape after completing placement of the inner pin 4, provided that the wires 1 are parts of a shape-memory alloy.

The inner pin 4 is a piece of a metal such as stainless steel or a piece of resin, coated with Teflon (registered trademark). In particular, the inner pin 4 is of a metal such as stainless steel, brass and copper or a resin such as polytetrafluoroethylene and polyacetal. Further, it is preferred to provide the surface of the inner pin 4 with a lubricating coating of polytetrafluoroethylene to make it easy to release the inner pin from the filter.

Then, the fixing core member 5 is inserted (pierced) into the wire assembly to constitute the aforesaid fixed assembly 10. The resultant fixed assembly 10 is immersed in a filter material solution. After completing formation of the filter membrane, the fixing core member 5 is extracted from the fixed assembly and the inner pin 4 is removed from the assembly. The inner pin 4 is removed through the gap between parts of the wires uncovered with the filter membrane. In this case, it is vital to ensure that the inner pin is extracted slowly and carefully since forcible extraction of the inner pin causes a fear of damaging the produced filter membrane during extraction. By such handling of the product, the fitter membrane that has been already provided on the wire assembly is prevented from being damaged in spite of the fact that the filter membrane is stretched to some extent during extraction of the inner pin since the membrane per se is of polyurethane and thus has extensibility.

The fixing core member 5 is used for setting the wire assembly 2 having inner pin 5 inserted therein on the fixed assembly holder 6 of the dipping apparatus 11. A preferred material for the fixing core member includes metals such as stainless steel, brass and copper, and resin such as polytetrafluoroethylene and polyacetal.

As described above, the method of producing filters for thrombus capture catheters according to the invention comprises: arranging a plurality of wires 1 so as to run helically, thereby forming a spindle-shaped wire assembly which is expanded at a middle portion 2C and which is converged at distal portion 2A and proximal portion 2B; immersing a predetermined part of said wire assembly 2 in a filter material solution so that said assembly 2 is covered with and fixed by said filter material; and then drying the resultant wire assembly 2 to form a filtering section 3. Thus, the filter produced by the above method has a filtering section composed of a parachute-shaped membrane which coverts the wires arranged in a circle.

For this reason, the filter for thrombus capture catheters comprises filtering section 3 made of a thin-walled membrane, but it becomes a filter with a sufficient strength since the filtering section 3 is reinforced by plural wires 1.

As the filter for thrombus capture catheters is constructed as described above, it is possible to produce the filters with a reduced diameter, which in turn makes it possible to reduce a size of delivery sheath, thus making it possible to alleviate both physical and mental burdens borne by patients to be catheterized. Further, the catheter is improved in capability to pass through the diseased area of the blood vessel.

## Claims

1. A method of producing filters configured for thrombus capture catheter, comprising the steps of:
arranging a plurality of wires (1) so as to run helically, thereby forming a spindle-shaped wire assembly (2) which is expanded at a middle portion (2C) and which is converged at distal and proximal portions (2A, 2B);
immersing a predetermined part of said wire assembly (2) in a filter material solution so that said assembly (2) is covered with and fixed by said filter material; and then
drying the resultant wire assembly to form a filtering section (3),
**characterized in that** the method further comprises the steps of:
arranging a half spindle-shaped inner pin (4) in an inside of said wire assembly (2);
inserting a core member (5) into the spindle-shaped wire assembly (2) to allow it to pass through the distal and proximal portions (2A, 2B) thereof and a central part of the inner pin (4);
dipping a pin-applied part of said wire assembly (2) in said filter material solution to form said filtering section (3); and
removing said core member (5) and said inner pin (4) after drying said dip-coated portion to complete a filter.

2. The method of producing filters configured for thrombus capture catheter according to claim 1, wherein said wire (1) is of a shape memory alloy, and wherein said filter material is a biocompatible synthetic resin.

3. The method of producing filters configured for thrombus capture catheter according to claim 1 or 2, wherein said inner pin (4) and core member (5) are coated with a lubricating film of a polytetrafluoroethylene. 1

## Patentansprüche

1. Verfahren zur Herstellung von Filtern, die für Thrombi auffangende Katheter konfiguriert sind, das die Schritte aufweist:
Anordnen mehrerer Drähte (1), so dass sie spiralförmig verlaufen, wodurch eine spindelförmige Drahtanordnung (2) ausgebildet wird, die in einem mittleren Abschnitt (2C) expandiert und an distalen und proximalen Abschnitten (2A, 2B) konvergiert;
Eintauchen eines vorgegebenen Teils der Drahtanordnung (2) in eine Filtermateriallösung, so dass die Anordnung (2) mit dem Filtermaterial überzogen und dadurch fixiert wird; und dann
Trocknen der sich ergebenden Drahtanordnung, um einen Filterabschnitt (3) zu bilden,
**dadurch gekennzeichnet, dass** das Verfahren ferner die folgenden Schritte aufweist:
Anordnen eines halben spindelförmigen Innenstifts (4) in einem Inneren der Drahtanordnung (2):
Einsetzen eines Kernelements (5) in die spindelförmige Drahtanordnung (2), um zuzulassen, dass es durch ihre distalen und proximalen Abschnitte (2A, 2B) und einen mittleren Teil des Innenstifts (4) geht;
Tauchen eines Teils mit angebrachtem Stift der Drahtanordnung (2) in die Filtermateriallösung, um den Filterabschnitt (3) auszubilden; und
Entfernen des Kernelements (5) und des Innenstifts (4) nach dem Trocknen des tauchbeschichteten Abschnitts, um einen Filter fertigzustellen.

2. Verfahren zur Herstellung von Filtern, die für Thrombi auffangende Katheter konfiguriert sind, nach Anspruch 1, wobei der Draht (1) aus einer Formgedächtnislegierung ist, und wobei das Filtermaterial ein biologisch verträgliches Kunstharz ist.

3. Verfahren zur Herstellung von Filtern, die für Thrombi auffangende Katheter konfiguriert sind, nach Anspruch 1 oder 2, wobei der Innenstift (4) und das Kernelement (5) mit einem Schmierfilm aus einem Polytetrafluorethylen überzogen sind.

## Revendications

1. Procédé destiné à produire des filtres configurés pour un cathéter de capture de thrombus, comprenant les étapes consistant à :
- agencer une pluralité de fils (1) de façon à ce qu'ils soient disposés de manière hélicoïdale, en formant de ce fait un ensemble filaire fusiforme (2) qui s'expanse au niveau d'une partie médiane (2C), et qui converge au niveau des parties distale et proximale (2A, 2B) ;
- immerger une partie prédéterminée dudit ensemble filaire (2) dans une solution de matériau de filtre de telle sorte que ledit ensemble (2) soit recouvert et fixé par ledit matériau de filtre ;
et ensuite :
- sécher l'ensemble filaire résultant de façon à former une section filtrage (3) ;
**caractérisé en ce que** le procédé comprend en outre les étapes consistant à :
- agencer une broche intérieure semi-fusiforme (4) à l'intérieur dudit ensemble filaire (2) ;
- insérer un élément de noyau (5) dans l'ensemble filaire fusiforme (2) de façon à lui permettre de passer à travers les parties distale et proximale (2A, 2B) de celui-ci, et une partie centrale de la broche intérieure (4) ;
- immerger une partie appliquée sur la broche dudit ensemble filaire (2) dans ladite solution de matériau de filtre de façon à former ladite section filtrage (3) ; et
- retirer ledit élément de noyau (5) et ladite broche intérieure (4) après avoir séché ladite partie recouverte par immersion de façon à réaliser un filtre.

2. Procédé destiné à produire des filtres configurés pour un cathéter de capture de thrombus selon la revendication 1, dans lequel ledit fil (1) est réalisé dans un alliage à mémoire de forme, et dans lequel ledit matériau de filtre est une résine synthétique biocompatible.

3. Procédé destiné à produire des filtres configurés pour un cathéter de capture de thrombus selon la revendication 1 ou la revendication 2, dans lequel ladite broche intérieure (4) et ledit élément de noyau (5) sont recouverts d'une pellicule lubrifiante réalisée en polytétrafluoroéthylène.
